# EUROPEAN PATENT SPECIFICATION

(11) **EP 2 266 719 B1**
(45) Date of publication and mention of the grant of the patent: **08.06.2016**
(21) Application number: 10010918.0
(22) Date of filing: 23.04.2002
(51) Int. Cl.: B09B 3/00, A61K 9/70, A61F 13/02

(54) **Disposal system for transdermal dosage form**
Entsorgungssystem für transdermale Dosierformen
Système jetable pour une forme galénique transdermique

(30) Priority: 23.04.2001 US 285862 P; 22.05.2001 US 292601 P
(43) Date of publication of application: 29.12.2010
(62) Divisional of application: 09010451.4
(73) Proprietor: EURO-CELTIQUE S.A., 1653 Luxembourg (LU)
(72) Inventor: Marcenyac, Geraldine, Nowalk, CT 06851 (US); Alfonso, Mark, Purcell, OK 73080 (US); Gerhlein, Lane, Naples, Florida 34109 (US); Howard, Stephan, Danbury, CT 06811 (US); Tavares, Lino, Kinneton, NJ 07405 (US); Greenbaum, Ronald, Rye Brook, NY 10573 (US); Levy, Seth, Danbury, CT 06811 (US); Oshlack, Benjamin, Boca Raton, FL 33432 (US)
(74) Representative: Maiwald, Walter

(56) References cited:
- EP-A2- 0 455 326
- EP-A2- 0 635 262
- WO-A1-95/16479
- US-A- 5 149 538
- US-A- 5 287 960
- US-A- 5 804 215

## Description

### Cross Reference to Related Applications

This application claims priority from U.S. Provisional Application Serial No. 60/285,862, filed April 23, 2001, and from U.S. Provisional Application Serial No. 60/292,601, filed May 22, 2001, published as US-A1- 2015/0182474.

### FIELD OF THE INVENTION

This invention relates to a tamper-resistant article of manufacture that can be used to prevent misuse of a transdermal dosage form. The article can contain a transdermal dosage form, for example a transdermal system, and includes a detection and/or inactivating agent that is released when the article or dosage form is misused, and/or a heat-sensitive adhesive that changes color when subjected to elevated temperatures and seals the transdermal dosage form within the article.

### BACKGROUND OF THE INVENTION

Transdermal dosage forms are convenient dosage forms for delivering many different active therapeutically effective agents, including but not limited to analgesics, such as opioid analgesics. Typical opioid analgesics include, but are not limited to, fentanyl, buprenorphine, etorphines, and other high potency narcotics. Typical non-opioid drugs are anti-emetics (scopolamine), cardiovascular agents (nitrates and clonidine) and hormones (estrogen and testosterone).

The most common transdermal dosage form is a diffusion-driven transdermal system (transdermal patch) using either a fluid reservoir or a drug-in-adhesive matrix system. Other transdermal dosage forms include, but are not limited to, topical gels, lotions, ointments, transmucosal systems and devices, and iontophoretic (electrical diffusion) delivery system.

Transdermal dosage forms are particularly useful for timed-release and sustained-release of active agents. However, many dosage forms, and particularly those for timed and sustained release of active agent(s), contain large amounts of active agent(s), often many times the actual absorbed dose. Often, the dosage form contains an excess of active agent or delivers less than the entire amount of its active agent to the subject being treated. This results in most of the active agent remaining in the dosage form after use. Both the unused dosage form and the portion of active agent that remains in the dosage form after use is subject to potential illicit abuse, particularly if the active agent is a narcotic or a controlled substance. For example, used dosage forms containing excess or unused opioids may be tampered with by chewing or extraction by a drug abuser. Even careful disposal of used dosage forms may not be completely effective in preventing abuse, particularly in cases of incomplete or partial compliance.

U.S. Patent No. 5,804,215 to Cubbage et al. ("Cubbage") relates to a disposal system for a transdermal patch comprising a pouch for transport of the patch. The disposal system encapsulates a transdermal patch and prevents access to it.

U.S. Patent No. 5,149,538 to Granger et al. ("Granger") relates to a misuse-resistive dosage form for the transdermal delivery of opioids.

There is a need for a transdermal dosage form or for a packaging that is less susceptible to abuse than is presently known in the art.

### SUMMARY OF THE INVENTION

Thus, it is an object of the present invention to provide an article that may be used in combination with a transdermal dosage form, *e*.*g*., a transdermal patch, wherein the article includes a reservoir housing a dye and/or medicament inactivating agent which is released when the reservoir is opened or revealed and/or a heat activated adhesive that changes color and is activated when the article is subjected to elevated temperatures, thereby sealing the transdermal dosage form within the article. This object is achieved by the disposed system in accordance with claim 1. Therefore, the article prevents or hinders misuse of the active agent contained in the transdermal dosage form.

The present diclosure provides a disposable article including:
(A) an outer layer having an inner layer-facing side and an opposite side;
(B) an inner layer having an outer layer-facing side and an opposite side, the inner layer joined to the outer layer to form one or more closed material reservoir between the outer layer-facing side of the inner layer and the inner layer-facing side of the outer layer; and
(C) adhesive covering a first portion of the opposite side of the inner layer, wherein the adhesive is a heat activated adhesive that changes color and/or is activated when the article is subjected to elevated temperatures, thereby sealing the transdermal dosage form within the article.

In a preferred embodiment, the article may include a pocket having a sealable opening and formed between first and second portions of the opposite side of the inner layer, wherein the opening is optionally sealed by a flap covered at least in part by a permanent pressure and/or heat sensitive adhesive. Still further, the article may also include a peelable release layer covering the adhesive. The adhesion of the adhesive to the opposite side of the outer layer is typically greater than the strength of the joining of the outer layer to the inner layer.

It is also an object to provide a method of disposing of a transdermal patch that includes placing a transdermal patch within the article described above, sealing the patch within a pocket of the article, and subjecting the article to elevated temperatures, such that the heat-activated adhesive is activated, and optionally changes color, and seals the transdermal patch within the article.

Alternatively, the present disclosure provides a disposable article including:
(A) an outer layer having an inner layer-facing side and an opposite side;
(B) an inner layer having an outer layer-facing side and an opposite side, the inner layer joined to the outer layer to form one or more closed material reservoir between the outer layer-facing side of the inner layer and the inner layer-facing side of the outer layer;
(C) a detection material and/or medicament inactivating agent in the reservoir which is released when the reservoir is opened or revealed; and
(D) adhesive covering a first portion of the opposite side of the inner layer.

In a preferred embodiment, the detection material is an indelible dye, each of the outer and inner layers are tear-resistant, and the layers are joined with a pressure sensitive adhesive. Further, the article may include a pocket having a sealable opening and formed between first and second portions of the opposite side of the inner layer, wherein the opening is optionally sealed by a flap covered a least in part by a permanent pressure and/or heat sensitive adhesive. Still further, the article may also include a peelable release layer covering the adhesive. The adhesion of the adhesive to the opposite side of the outer layer is typically greater than the strength of the joining of the outer layer to the inner layer.

It is also an object to provide a method of disposing of a transdermal patch that includes placing a transdermal patch within the article described above and sealing the patch within a pocket of the article, such that the article releases the detection material and/or inactivating agent when the reservoir is opened.

Further, the present disclosure provides an article including:
(A) a medicament layer having a transfer side and an opposite side;
(B) a first adhesive contained in the medicament layer or covering at least a portion of the transfer side of the medicament layer;
(C) an outer layer having a medicament layer-facing side and an opposite side, the medicament layer being joined to outer layer to form one or more closed material reservoir between the opposite side of the medicament layer and the medicament layer-facing side of the outer layer; and
(D) a detection material and/or medicament-inactivator in the reservoir which is released when the reservoir is opened.

Preferably, the medicament layer contains an opiate, e.g., fentanyl; the detection material is an indelible dye; each of the outer and inner layers are tear-resistant; and the layers are joined with a pressure and/or heat sensitive adhesive. Further, the article may also include a peelable release layer covering the adhesive, and the adhesion of the adhesive to itself or the opposite side of the outer layer is greater than the strength of the joining of the outer layer to the medicament layer.

Still further, the present disclosure provides a method of disposing of the above article by folding the article so that opposite sides of the medicament layer are permanently sealed by the second adhesive.

Finally, the present disclosure provides an article having a transfer side and an opposite side, wherein the transfer side includes a first region and a second region adjacent to the first region. The first and second regions are separated by a membrane. The first region has a detection and/or inactivating material disposed therein, and the second region has a medicament disposed therein. The article may also contain an adhesive covering at least a portion of the perimeter of the transfer side, and the adhesive is optionally a heat-sensitive adhesive.

Therefore, the present disclosure provides an article comprising:
(A) an outer layer having an inner layer-facing side and an opposite side;
(B) an inner layer having an outer layer-facing side and an opposite side, said inner layer joined to said outer layer to form one or more closed material reservoir between said outer layer-facing side of said inner layer and said inner layer-facing side of said outer layer;
(C) one or more materials in said reservoir which are activated or released when said reservoirs are activated, wherein said materials are selected from the group consisting of a detection material, an inactivating agent, or a combination thereof; and
(D) adhesive covering a first portion of said opposite side of said inner layer, wherein the adhesive is optionally heat-sensitive.

The present disclosure further provides an article comprising:
(A) a tear-resistant outer layer having an inner layer-facing side and an opposite side;
(B) a tear-resistant inner layer having an outer layer-facing side and an opposite side, said inner layer joined to said outer layer to form one or more closed material reservoir between said outer layer-facing side of said inner layer and said inner layer-facing side of said outer layer;
(C) one or more materials in said reservoirs which are activated or released when said reservoirs are activated, wherein said materials are selected from the group consisting of a detection material, an inactivating agent, or a combination thereof;
(D) a permanent, pressure and/or heat sensitive adhesive covering a first portion of the perimeter of said opposite side of said inner layer; and
(E) a peelable release layer covering said adhesive;
wherein the adhesion of (1) said adhesive to itself or to said opposite side of said inner layer is greater than the strength of (2) the joining of said outer layer to said inner layer.

The present disclosure additionally provides a method of disposing of a transdermal patch, said method comprising:
(A) placing said transdermal patch on said opposite side of said inner layer of an article as described above; and
(B) contacting said adhesive covering at least a portion of said opposite side of said inner layer with a different portion of said opposite side of said inner layer to form a sealed pocket enclosing said transdermal patch.

The present disclosure also provides an article comprising:
(A) an outer layer having an inner layer-facing side and an opposite side;
(B) an inner layer having an outer layer-facing side and an opposite side, said inner layer being joined to said outer layer, to form one or more closed material reservoir between outer layer-facing side of said inner layer and said inner layer-facing side of said outer layer, and wherein a first portion of said opposite side of said inner layer is joined to a second portion of said opposite side of said inner layer, to form a pocket between first and second portions of said opposite side of said inner layer, said pocket having a sealable opening; and
(C) one or more materials in said reservoirs which are activated or released when said reservoirs are activated, wherein said materials are selected from the group consisting of a detection material, an inactivating agent, or a combination thereof.

The present disclosure further provides an article comprising:
(A) a tear-resistant outer layer having an inner layer-facing side and an opposite side;
(B) a tear-resistant inner layer having an outer layer-facing side and an opposite side,
   said inner layer being joined to said outer layer to form one or more closed material reservoirs between said outer layer-facing side of said inner layer and said inner layer-facing side of said outer layer, and
   a first portion of said opposite side of said inner layer joined to a second portion of said opposite side of said inner layer, to form a pocket between first and second portions of said opposite side of said inner layer;
(C) one or more materials in said reservoirs which are released when said reservoir are opened, wherein said materials are selected from the group consisting of a detection material, an inactivating agent, or a combination thereof;
(D) a sealable flap adapted to seal said opening;
(E) a permanent pressure and/or heat sensitive adhesive covering at least a portion of said flap; and
(F) a peelable release layer covering said adhesive;
wherein the adhesion of (1) said adhesive to said opposite side of said outer layer is greater than the strength of (2) the joining of said outer layer to said inner layer.

The present disclosure also provides a method of disposing of a transdermal patch, said method comprising:
(A) placing said in said pocket of an article as described above; and
(B) sealing said pocket to form a sealed pocket enclosing said transdermal patch.

The present disclosure further provides an article comprising:
(A) a medicament layer having a transfer side and an opposite side;
(B) a first adhesive contained in said medicament layer or covering at least a portion of said transfer side of said medicament layer, wherein the adhesive is optionally heat sensitive;
(C) an outer layer having a medicament layer-facing side and an opposite side, said medicament layer being joined to said outer layer, to form one or more closed material reservoir between said opposite side of said medicament layer and said medicament layer-facing side of said outer layer; and
(D) one or more materials in said reservoir which are activated or released when said reservoir is activated or opened, wherein said materials are selected from the group consisting of a detection material, an inactivating agent, or a combination thereof.

Alternatively, the present disclosure provides an article comprising:
(A) a tear-resistant medicament layer having an medicament transfer side and an opposite side and containing an opiate;
(B) a first pressure and/or heat sensitive adhesive contained in or covering at least a portion of said medicament transfer side of said medicament layer;
(C) a second pressure and/or heat sensitive adhesive, which may the same as or different from said first adhesive and which is permanent, covering at least a portion of the peri-perimeter of said medicament layer.
(D) a tear-resistant outer layer having a medicament layer-facing side and an opposite side, said medicament layer being joined to said outer layer to form one or more closed material reservoir between said opposite side of said medicament layer and said medicament layer-facing side of said outer layer;
(E) one or more materials in said reservoir which are released when said reservoirs are opened, wherein said materials are selected from the group consisting of a detection material, an inactivating agent, or a combination thereof;
(F) a first peelable release layer covering said first adhesive; and
(G) a second peelable release layer covering said second adhesive;
wherein said second adhesive is prevented from adhering to skin while said first adhesive is adhering to said skin, and
wherein the adhesion of (1) said second adhesive to itself or to said opposite side of said outer layer is greater than the strength of (2) the joining of said outer layer to said medicament layer.

The present disclosure further provides a method of disposing of an article, said method comprising:
(A) folding an article as described above so that said opposite side of said medicament layer is sealed with said second adhesive.

In an additional embodiment, the present disclosure provides an article comprising:
(A) a transfer side and an opposite side wherein the transfer side comprises a first region and a second region adjacent to said first region, wherein said first and second regions are separated by a membrane;
(B) one or more materials disposed in said first region selected from the group consisting of a detection material, and inactivating agent, or a combination thereof, wherein said materials are activated or released when said first region is folded onto said second region;
(C) a medicament disposed in said second region.

The article described above may contain one or more of the following features: the inner and outer layers are tear-resistant; the material in the material reservoir is selected from the group consisting of an indelible dye, a biological inactivating agent, a chemical inactivating agent, a denaturing inactivating agent, an electrical inactivating agent, a magnetic inactivating agent, a mechanical inactivating agent, a cross-linking inactivating agent, or any combination of any of the foregoing; the reservoir is activated by application of pressure, cutting, opening, puncturing, sealing, tearing, unsealing, or any combination of any of the foregoing; the inactivating agent is selected from the group consisting of rat or human mu-opioid receptor, opioid neutralizing antibodies, a narcotic antagonist, irritating or dipphoric agents, or any combination thereof; the adhesive comprises a permanent, pressure and/or heat sensitive adhesive; the first portion of said opposite side of said inner layer comprises at least a portion of the perimeter of said opposite side of said inner layer; the article further comprises (E) a release layer covering said adhesive, wherein said release layer is optionally peelable; the adhesion of (1) said adhesive to itself or to said opposite side of said inner layer is greater than the strength of (2) the joining of said outer layer to said inner layer.

Finally, the present disclosure provides a container for a transdermal dosage form including (a) one or more material reservoirs sized to receive a transdermal dosage form, and (b) an adhesive capable of sealing a transdermal dosage form within the reservoir, wherein the adhesive is a heat activated adhesive that changes color and/or is activated when the article is subjected to elevated temperatures, thereby sealing the transdermal dosage form within the container. The container of the present disclosure may further include a flap that is covered, at least in part, by a permanent pressure and/or heat sensitive adhesive, and optionally the container may also include a peelable release layer covering the adhesive. Still further, the container may include a detection material and/or medicament-inactivating agent in communication with the reservoir that is released when the reservoir is opened or revealed. The container of the present disclosure may also be tear-resistant and it may further comprise a medicament layer. Optionally, the detection material or inactivating material is housed in a first reservoir and the container also includes a second reservoir that houses a medicament, wherein the first and second reservoirs are in communication, such that if the container is opened or revealed, the contents of the first reservoir are released and mix with the contents of the second reservoir.

### BRIEF DESCRIPTION OF THE DRAWINGS

Figures 1a-1d show an article having a pouch for insertion of a transdermal patch in accordance with the present invention.
Figure 2 shows a transdermal dosage form containing both a medicament and a detection and/or inactivating agent
Figure 3 shows a transdermal dosage from having a detection and/or inactivating agent in a first region and a medicament in a second region.
Figure 4a-4d shows an article having a pouch for insertion of a transdermal patch in accordance with the present invention.

### DETAILED DESCRIPTION

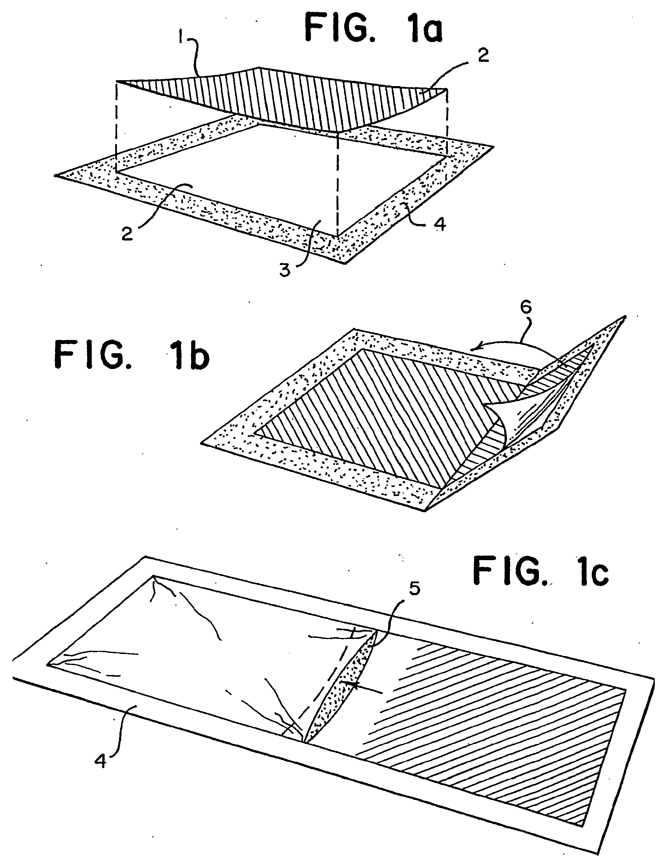

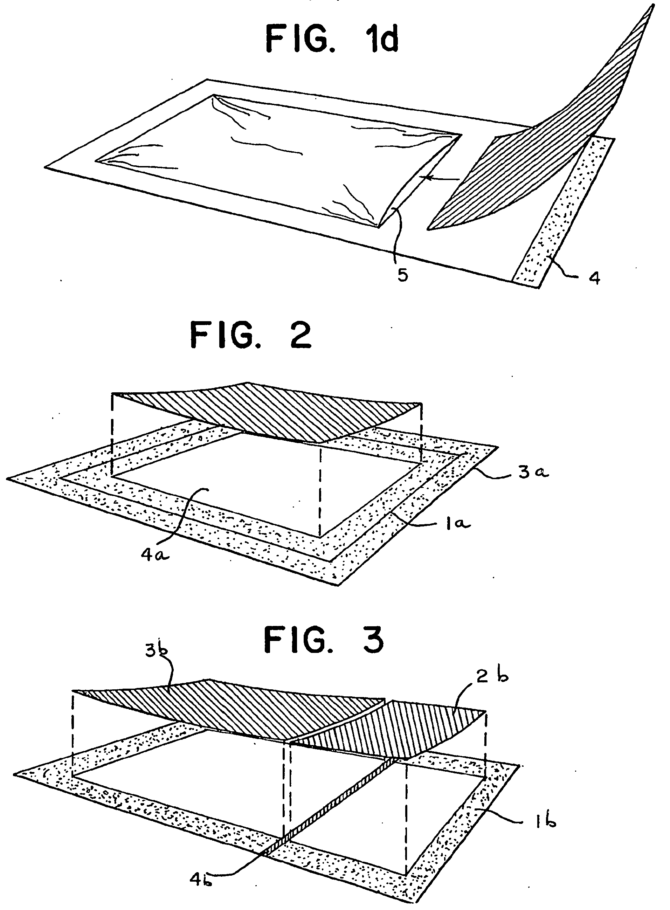

Figure 1 illustrates one embodiment of an article of the present invention. As shown in Figure 1(a), this article includes an outer layer (1) and an inner layer (3), which are joined by an adhesive (4) covering a first portion of the inner layer. The combination of the inner and outer layers forms a closed detection material and/or inactivator reservoir (2) between the outer layer facing-side of the inner layer and the inner layer-facing side of the outer layer. As shown in Figures 1(b-d), the inner and outer layers are preferably joined in such a way as to form a sealable opening between the two layers. The opening (5) is preferably sealed by a flap (6) containing an adhesive (4) covering at least a portion of the flap that is used to seal the opening. Further, as shown in Figures 1(b-d), a first portion of the opposite side of the inner layer of the article may include at least a portion of the perimeter of the opposite side of the outer layer.

Optionally, a release layer covers the adhesive, and preferably the release layer is peelable. In a preferred embodiment, the adhesion of the adhesive to itself or to the opposite side of the inner layer is greater than the adhesive strength of the joining of the outer layer to the inner layer. Preferably, each of the layers of the articles of the invention are tear-resistant.

According to the invention, the perimeter of the detection material and/or inactivator reservoir has a thickness in the range of 12-100 microns, and the center of the detection material and/or inactivator reservoir is thinner than the perimeter of the reservoir. The differential in thickness of the perimeter and center of the reservoir causes the inactivator to be released when the article is folded or the patch is removed from the article.

The article described above and illustrated in Figure 1 may be used to house a transdermal dosage form. In practice, the detection material and/or inactivator is released when the reservoir is opened, and optionally either one or both of these materials may contact and/or penetrate the transdermal dosage form, optionally altering, damaging, and/or inactivating the medicament contained within.

Figure 2 illustrates an alternate embodiment. The article is a transdermal dosage form having a detection and/or inactivator material disposed within the dosage form in addition to a medicament Specifically, the article includes a medicament layer (1a) having a transfer side and an opposite side, as well as a first adhesive disposed on the medicament layer or covering a portion of the transfer side. The article further includes an outer layer (3a) having a medicament layer-facing side and an opposite side, wherein the medicament layer is joined to the outer layer to form a closed detection material and/or inactivator reservoir (4a) between the opposite side of the medicament layer and the medicament layer-facing side of the outer layer. Preferably, each of the layers of the article of the invention are tear-resistant.

The article optionally includes a release layer covering the adhesive. Further, the release layer is optionally peelable, and the adhesion of the adhesive to itself or to the opposite side of the outer layer is greater than the adhesive strength of the joining of the outer layer to the medicament layer. The article may also include a second adhesive, which is the same or different from the first adhesive, wherein the second adhesive covers at least a portion of the peri-perimeter of the medicament layer. Preferably, the second adhesive is prevented from adhering to the medicament-receiving surface, i.e, the skin, while the first adhesive is adhering to the skin. If a second adhesive is included in the article, it optionally contains a second release layer, which may also be peelable, and the adhesion of the second adhesive to itself or to the opposite side of the outer layer is greater than the adhesive strength of the joining of the outer layer to the medicament layer.

The article may also optionally contain an additional adhesive layer contained within the inner layer or pouch so that a transdermal patch placed within the article will be held within the article. The additional adhesive layer optionally includes a release layer covering the adhesive, and the release layer is optionally peelable.

Figure 3 illustrates an additional embodiment. The article is a transdermal dosage form having a transfer side and an opposite side, and optionally an adhesive (1b) disposed on the periperimeter of the transfer side. The transfer side comprises a first (2b) and second (3b) region, wherein the regions are separated from one another by a membrane or impermeable barrier (4b). The first region has a detection and/or inactivator material disposed therein. The second region has a medicament disposed therein. The article optionally includes a release layer covering the adhesive, and the release layer is optionally peelable.

In practice, the inactivating agent is contacted with the medicament when the patch is folded such that the first region contacts the second region, optionally altering, damaging and/or inactivating the medicament contained within.

The inactivating agent, when contacted with or a medicament or active agent to be placed in the article or when activated itself, renders the active agent unavailable through inactivation, such as for example chemical inactivation or alteration of the receptor binding site of the active agent; electrical inactivation or alteration of the receptor binding site of the active agent; magnetic inactivation or alteration of the receptor binding site of the active agent; mechanical inactivation or alteration of the receptor binding site of the active agent; biounavailability; physical unavailability; loss of appeal of the active agent to the abuser, such as for example, an inactivating agent which creates an intolerably bad taste or an intolerable reaction such as extreme nausea or the like; or something similar thereto. One or more inactivating agent(s) may be used.

For example, if the active agent in a transdermal patch to be disposed of by placing it in the present article were an opioid, the inactivating agent could be a chemical or denaturing agent that would alter residual opioid molecules in the dosage form and make them inactive. The inactivating agent could be an opioid receptor that would bind the residual opioid into an insoluble ligand-receptor complex. The inactivating agent could also be an opioid receptor antagonist, preferably with greater specificity and/or affinity for the receptor than the opioid, which would be isolated or delivered with the residual opioid upon misuse and compete with the residual opioid for the opioid receptor, thereby defeating the purpose of misusing the opioid. This would render the residual opioid useless *in vivo.* The inactivating agent could also physically sequester the residual active agent such as, for example, in an impermeable microsphere or in a permanently bound matrix. Similarly, the inactivating agent could be a non-opioid with distressing or dysphoric properties if absorbed that made abuse unappealing.

The reservoir could be opened, revealed or activated by, for example, application of pressure, cutting, opening, puncturing, sealing, tearing, unsealing, or any combination of any of the foregoing.

In practice, the detection material and/or inactivator is preferably released when the reservoir is opened or revealed, and optionally either one or both of these materials may contact and/or penetrate the medicament layer, optionally altering, damaging, and/or inactivating the medicament contained within.

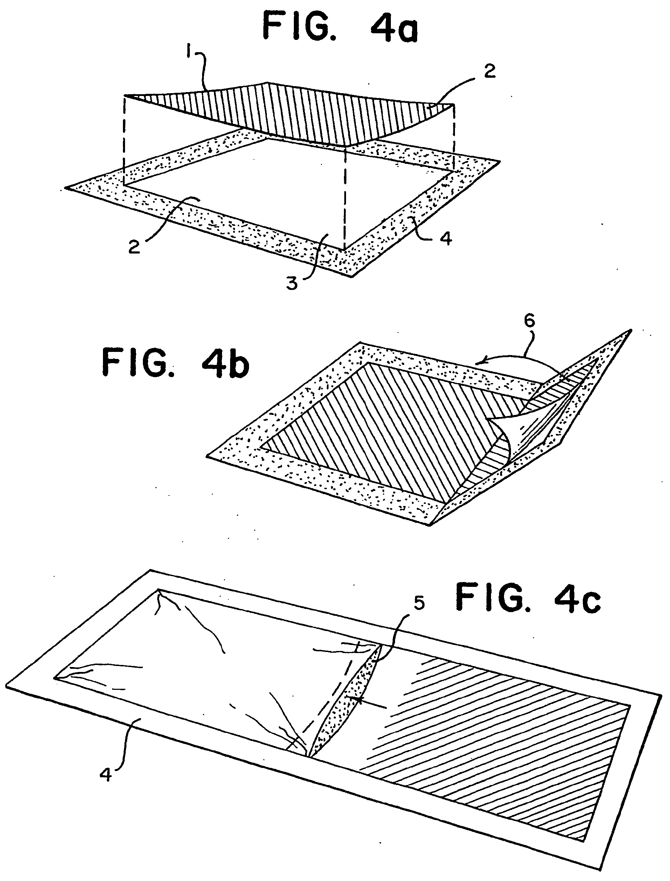

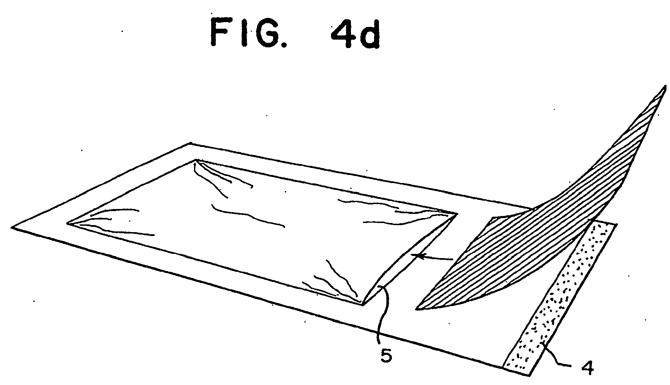

Figure 4 illustrates a further embodiment of an article of the present invention. As shown in Figure 4(a), this article includes an outer layer (1) and an inner layer (3), which are joined by a heat sensitive adhesive (4) covering a first portion of the inner layer. The combination of the inner and outer layers forms a closed reservoir (2) between the outer layer facing-side of the inner layer and the inner layer-facing side of the outer layer, which contains a detection and/or inactivating material. As shown in Figures 4(b-d), the inner and outer layers are preferably joined in such a way as to form a sealable opening between the two layers. The opening (5) is preferably sealed by a flap (6) containing a heat sensitive adhesive (4) covering at least a portion of the flap that is used to seal the opening. Further, as shown in Figures 4(b-d), a first portion of the opposite side of the inner layer of the article may include at least a portion of the perimeter of the opposite side of the outer layer.

Optionally, a release layer covers the adhesive, and preferably the release layer is peelable. In a preferred embodiment, the adhesion of the adhesive to itself or to the opposite side of the inner layer is greater than the adhesive strength of the joining of the outer layer to the inner layer. Preferably, each of the layers of the articles of the invention are tear-resistant.

According to the invention, the perimeter of the reservoir has a thickness in the range of 12-100 microns, and the center of the reservoir is thinner than the perimeter of the reservoir.

The article described above and illustrated in Figure 4 may be used to house a transdermal dosage form. In practice, the heat-sensitive adhesive is activated when the article is exposed to elevated temperature, e.g., at or above 100°C, and seals the reservoir containing the transdermal dosage form. Optionally, the adhesive also changes color when activated.

One or more of the elements of the article of the invention are contained within a portion of the layers of the article. The term *"portion"* refers to all or any part of the layer. Further, certain of the elements of the article are located on or proximate to the periperimeter of the article or a layer of the article. The term "*periperimeter*" refers to the perimeter or an area close to the perimeter.

The detection material used in the article of the invention is preferably a dye, and more preferably an indelible dye. Further, the adhesive materials used in the article of the invention are preferably permanent, pressure and/or heat sensitive adhesives. The adhesives used in the article of the invention may be same or different. Preferably, the adhesives are chosen so as to insure that the adhesion of the adhesive to itself or to the opposite side of the inner or outer layer is greater than the adhesive strength of the joining of the outer layer to the inner or medicament layer. If the same adhesive is used throughout the article, the preferred variations in adhesive strength may be achieved e.g., by varying the amount or concentration of the adhesive in a particular portion of the article.

The layers of the article of the invention are joined by any technique known to those skilled in the art. For example, the layers may be joined by an adhesive or by heat-sealing the layers to one another. The method of joining the layers is chosen in order to avoid interference with the proper functioning of the pouch, namely to release dye or inactivator upon misuse. The layers may originate from a single member that is folded on itself to form the inner and outer portions or the layers may originate from two individual members.

The article of the invention optionally includes a medicament inactivating agent The inactivating agent may be contained in the detector reservoir and mixed with the detection material, or disposed within a separate region of the detector reservoir reserved for inactivating agent and designed to prevent mixing of the detection material and inactivating agent The inactivating agent may be released when the dosage form is handled in a particular manner, such as when it is folded, bent, squeezed with sufficient force, peeled from the skin, or the like, or if the dosage form is abused, such as for example, is chewed, soaked, subjected to extraction, smoked, or the like. This embodiment could be used, for example, to inactivate any residual active agent when the dosage form is discarded.

The inactivating agent, when contacted with the active agent or when activated itself, renders the active agent unavailable through inactivation, such as for example chemical inactivation or alteration of the receptor binding site of the active agent; electrical inactivation or alteration of the receptor binding site of the active agent; magnetic inactivation or alteration of the receptor binding site of the active agent; mechanical inactivation or alteration of the receptor binding site of the active agent; biounavailability; physical unavailability; loss of appeal of the active agent to the abuser, such as for example, an inactivating agent which creates an intolerably bad taste or an intolerable reaction such as extreme nausea or the like; or something similar thereto. One or more inactivating agent(s) may be used in a dosage form, as well.

For example, if the active agent in the dosage form were an opioid, the inactivating agent could be a chemical or denaturing agent that would alter residual opioid molecules in the dosage form and make them inactive. The inactivating agent could be a biological inactivating agent such as, for example, an opioid receptor that would bind the residual opioid into an insoluble ligand-receptor complex. The inactivating agent could also be an opioid receptor antagonist, preferably with greater specificity and/or affinity for the receptor than the opioid, which would be isolated or delivered with the residual opioid upon misuse and compete with the residual opioid for the opioid receptor, thereby defeating the purpose of misusing the opioid. This would render the residual opioid useless *in vivo* when the two were administered or isolated together. The inactivating agent could also physically sequester the residual active agent such as, for example, in an impermeable microsphere or in a permanently bound matrix. Alternatively, he inactivating agent could be a cross-linking agent that physically sequesters residual active agent. Similarly, the inactivating agent could be a non-opioid with distressing or dysphoric properties if absorbed that made abuse unappealing.

The inactivating agent, when contacted with the active agent or when activated itself, renders the active agent unavailable through inactivation, such as for example chemical inactivation or alteration of the receptor binding site of the active agent; electrical inactivation or alteration of the receptor binding site of the active agent; magnetic inactivation or alteration of the receptor binding site of the active agent; mechanical inactivation or alteration of the receptor binding site of the active agent; biounavailability; physical unavailability; loss of appeal of the active agent to the abuser, such as for example, an inactivating agent which creates an intolerably bad taste or an intolerable reaction such as extreme nausea or the like; or something similar thereto. One or more inactivating agent(s) may be used in a dosage form, as well.

Non-limiting examples of active agents that may be used in the present invention are fentanyl, buprenorphine, etorphine and related opioids of sufficient potency to allow transdermal usage, or any combinations thereof. Non-limiting examples of inactivating agents include the rat or human mu-opioid receptor; opioid-neutralizing antibodies; narcotic antagonists such as naloxone, naltrexne and nalmefene; dysphoric or irritating agents such as scopolamine, ketamine, atropine or mustard oils; or any combinations thereof.

The amount of detection material contained within the article of the invention is that amount which, when released, will be visible to the end-user. Such amounts can easily be ascertained by the skilled artisan, based on the dimensions and permeability of the article, as well as the visibility of the detection material. Further, the amount of inactivating agent will depend upon the active agent and the amount of residual active agent that is expected in a particular dosage form. Such amounts can also be determined by those skilled in the art by methods such as establishing a matrix of amounts and effects. However, such amounts should be those amounts effective to achieve the results sought, i.e., inactivation of the residual active agent or the rendering undesirable of an attractive drug of abuse.

The transdermal dosage form is preferably in the form of a transdermal patch. The dosage form contains active agent, and optionally an inactivating agent Preferably, the active agent is a chemically or biologically active agent. Most preferably, the active agent is an opioid analgesic including, but not limited to, fentanyl, buprenorphine, etorphines, and other high potency narcotics, or a non-opioid drug including, but not limited to, anti-emetics (scopolamine), cardiovascular agents (nitrates & clonidine) and hormones (estrogen & testosterone). One or more active agent(s) may be used in a dosage form.

### EXAMPLES

The following examples serve to illustrate, rather than limit, the scope of the present invention.

### Example 1

Upon expiration of the proscribed term of use of a transdermal patch containing an opioid, the patch is peeled off the skin of the user and disposed of using a disposable article depicted in Figure 1(a). In utilizing this article, the patch is placed generally in the center of the inner layer. The inner layer is then covered with the outer layer, thereby encasing or sandwiching the used patch within a reservoir formed between the two layers. The inner and outer layers are held together by adhesive. The patch may also be held in place with a layer of additional adhesive coated on either the inner or outer layers, or both.

The disposal device also includes a detection material and/or inactivator reservoir. Once the used patch is encased in the disposal device and the layers are sealed, when the device is tampered with, e.g., torn, the detection material and/or inactivator reservoir releases an indelible ink that mixes with the contents of the device and contacts whomever is tampering with the disposal device, thereby providing a visible indication that the device has been tampered with and who has tampered with it.

### Example 2

Upon expiration of the proscribed term of use of a transdermal patch containing an opioid, the patch is peeled off the skin of the user and disposed of using a disposable article depicted in Figure 1(b). In utilizing this article, the patch is placed generally in the center of the inner layer and the outer layer is folded in on the inner layer, creating a pouch that sandwiches the used patch within the material reservoir formed between the inner and outer layers. An adhesive is used to secure the inner layer to the outer layer. The patch may also be held within the reservoir by adhesive contained within the material reservoir.

The disposal device also includes a detection material and/or inactivator reservoir. Once the used patch is encased in the disposal device and the layers are sealed, when the device is tampered with, e.g., torn, the detection material and/or inactivator reservoir releases an opioid receptor antagonist and a dye that mix with the contents of the device, thereby providing a material which will counter the activity of any opioid remaining in the used patch, and soiling the tampered and providing a visible indication that the device has been tampered with and who has tampered with it

### Example 3

Upon expiration of the proscribed term of use of a transdermal patch containing an opioid, the patch is peeled off the skin of the user and disposed of using a disposable article depicted in Figure 1(c). In utilizing this article, the patch is placed generally inside the pouch formed by the adhesion of the inner and outer layers of the device. The pouch is then sealed by positioning the flap of the article on top of the outer layer and adhering the flap to the outer layer with adhesive. The patch may also be held within the reservoir by adhesive contained within the pouch.

The disposal device also includes a detection material and/or inactivator reservoir. Once the used patch is encased in the disposal device and the pouch is sealed, when the device is tampered with, e.g., torn, the detection material and/or inactivator reservoir releases an opioid receptor antagonist and a dye that mix with the contents of the device, thereby providing a material which will counter the activity of any opioid remaining in the used patch, and soiling the tampered and providing a visible indication that the device has been tampered with and who has tampered with it.

### Example 4

Upon expiration of the proscribed term of use of a transdermal patch containing an opioid as depicted in Figure 2, the patch is peeled off the skin of the user and disposed of. The patch also includes a detection material and/or inactivator reservoir. When the used patch is tampered with, e.g., torn, the detection material and/or inactivator reservoir releases an indelible ink that mixes with the contents of the patch and contacts whomever is tampering with the patch, thereby providing and soiling the tampered and a visible indication that the used patch has been tampered with and who has tampered with it.

### Example 5

Upon expiration of the proscribed term of use of a transdermal patch containing an opioid as depicted in Figure 2, the patch is peeled off the skin of the user and folded so that the medicament layer is sealed within an envelope formed by the outer layer. The patch also includes a detection material and/or inactivator reservoir. When the used patch is tampered with, e.g., torn, the detection material and/or inactivator reservoir releases an opioid receptor antagonist that mixes with any residual medicament in the patch, thereby providing a material which will counter the activity of any opioid remaining in the used patch, and a dye that leaks from the reservoir and soiling the tampered and providing a visible indication that the device has been tampered with and who has tampered with it.

### Example 6

Upon expiration of the proscribed term of use of a transdermal patch containing an opioid, the patch is peeled off the skin of the user and disposed of using a disposable article depicted in Figure 4(a). In utilizing this article, the patch is placed generally in the center of the inner layer. The inner layer is then covered with the outer layer, thereby encasing or sandwiching the used patch within a reservoir formed between the two layers. The inner and outer layers are held together by heat sensitive adhesive. The patch may also be held in place with a layer of additional adhesive coated on either the inner or outer layers, or both. Once the used patch is encased in the disposal device, the layers are sealed by exposing the article to elevated temperature.

### Example 7

Upon expiration of the proscribed term of use of a transdermal patch containing an opioid, the patch is peeled off the skin of the user and disposed of using a disposable article depicted in Figure 4(b). In utilizing this article, the patch is placed generally in the center of the inner layer and the outer layer is folded in on the inner layer, creating a pouch that sandwiches the used patch within the material reservoir formed between the inner and outer layers. A heat sensitive adhesive is used to secure the inner layer to the outer layer. The patch may also be held within the reservoir by heat sensitive adhesive contained within the material reservoir. Once the used patch is encased in the disposal device, the layers are sealed by exposing the article to elevated temperature.

### Example 8

Upon expiration of the proscribed term of use of a transdermal patch containing an opioid, the patch is peeled off the skin of the user and disposed of using a disposable article depicted in Figure 1(c). In utilizing this article, the patch is placed generally inside the pouch formed by the adhesion of the inner and outer layers of the device. The pouch is then sealed by positioning the flap of the article on top of the outer layer and adhering the flap to the outer layer with heat sensitive adhesive. The patch may also be held within the reservoir by adhesive contained within the pouch. Once the used patch is encased in the disposal device, the layers are sealed by exposing the article to elevated temperature.

Further embodiments are the following:
1. An article comprising:
   (A) an outer layer having an inner layer-facing side and an opposite side;
   (B) an inner layer having an outer layer-facing side and an opposite side, the inner layer joined to the outer layer to form one or more closed material reservoir between the outer layer-facing side of the inner layer and the inner layer-facing side of the outer layer; and
   (C) adhesive covering a first portion of the opposite side of the inner layer, wherein the adhesive is a heat activated adhesive that changes color and/or is activated when the article is subjected to elevated temperatures, thereby sealing the transdermal dosage form within the article.
2. An article of item 1, wherein the article is disposable.
3. An article of item 1, wherein the article may include a pocket having a sealable opening and formed between first and second portions of the opposite side of the inner layer, wherein the opening is optionally sealed by a flap covered a least in part by a permanent pressure and/or heat sensitive adhesive.
4. An article of item 1, wherein the article may also include a peelable release layer covering the adhesive.
5. An article as defined in item 1, wherein the adhesion of (1) said adhesive to itself or to said opposite side of said outer layer is greater than the strength of (2) the joining of said outer layer to said inner layer.
6. A method of disposing of a transdermal patch, said method comprising:
   (A) sealing the patch within a pocket of the article defined in item 1; and
   (B) subjecting the article to elevated temperatures, such that the heat-activated adhesive is activated, and optionally changes color, and seals the transdermal patch within the article.
7. An article comprising:
   (A) an outer layer having an inner layer-facing side and an opposite side;
   (B) an inner layer having an outer layer-facing side and an opposite side, said inner layer joined to said outer layer to form a closed detection material reservoir between said outer layer-facing side of said inner layer and said inner layer-facing side of said outer layer;
   (C) a detection material in said reservoir which is released when said reservoir is opened; and
   (D) adhesive covering a first portion of said opposite side of said inner layer.
8. An article of item 7, wherein said detection material comprises an indelible dye.
9. An article of item 7, wherein said detection material is selected from the group consisting of a dye, a pigment, a fluorescent material, or any combination of any of the foregoing.
10. An article of item 7, wherein said inner and outer layers are tear-resistant.
11. An article of item 7, wherein said adhesive comprises a permanent pressure sensitive adhesive.
12. An article of item 7, wherein the article may include a pocket having a sealable opening and formed between first and second portions of the opposite side of the inner layer, wherein the opening is optionally sealed by a flap covered a least in part by a permanent pressure and/or heat sensitive adhesive.
13. An article as defined in item 7, further comprising: (E) a release layer covering said adhesive.
14. An article as defined in item 7, wherein said release layer is peelable.
15. An article as defined in item 7, wherein the adhesion of (1) said adhesive to the opposite side of the outer layer is greater than the strength of (2) the joining of the outer layer to the inner layer.
16. A method of disposing of a transdermal patch, said method comprising:
   (A) placing the patch within the article defined in item 7; and
   (B) sealing the patch within a pocket of the article, such that the article release the detection material and/or inactivating agent when the reservoir is opened.
17. An article comprising:
   (A) a medicament layer having a transfer side and an opposite side;
   (B) a first adhesive contained in the medicament layer or covering at least a portion of the transfer side of the medicament layer;
   (C) an outer layer having a medicament layer-facing side and an opposite side, the medicament layer being joined to outer layer to form one or more closed material reservoir between the opposite side of the medicament layer and the medicament layer-facing side of the outer layer; and
   (D) a detection material and/or medicament-inactivator in the reservoir which is released when the reservoir is opened.
18. An article of item 17, wherein the medicament layer contains an opiate.
19. An article of item 18, wherein the opiate is fentanyl.
20. An article of item 17, wherein the detection material is an indelible dye.
21. An article of item 17, wherein said inner and outer layers are tear-resistant.
22. An article of item 17, wherein said layers are joined with a pressure and/or heat sensitive adhesive.
23. An article of item 17, wherein the article may also include a peelable release layer covering the adhesive.
24. An article as defined in item 17, wherein the adhesion of (1) said adhesive to itself or to said opposite side of said outer layer is greater than the strength of (2) the joining of said outer layer to said medicament layer.
25. A method of disposing of a transdermal patch, wherein said method comprising folding the article defined in item 17 such that the opposite sides of the medicament layer are permanently sealed by the second adhesive.
26. An article comprising a transfer side and an opposite side, wherein the transfer side includes
   (A) a first region having a detection and/or inactivating material disposed therein; and
   (B) an adjacent a second region, wherein the second region contains medicament disposed therein; and
   (C) first and second sections are separated by a membrane.
27. An article of item 26, wherein the article further contains an adhesive covering at least a portion of the perimeter of the transfer side, and the adhesive is optionally a heat-sensitive adhesive.
28. An article comprising
   (A) an outer layer having an inner layer-facing side and an opposite side;
   (B) an inner layer having an outer layer-facing side and an opposite side, said inner layer joined to said outer layer to form one or more closed material reservoir between said outer layer-facing side of said inner layer and said inner layer-facing side of said outer layer;
   (C) one or more materials in said reservoir which are activated or released when said reservoirs are activated, wherein said materials are selected from the group consisting of a detection material, an inactivating agent, or a combination thereof; and
   (D) adhesive covering a first portion of said opposite side of said inner layer, wherein the adhesive is optionally heat-sensitive.
29. An article comprising:
   (A) a tear-resistant outer layer having an inner layer-facing side and an opposite side;
   (B) a tear-resistant inner layer having an outer layer-facing side and an opposite side, said inner layer joined to said outer layer to form one or more closed material reservoir between said outer layer-facing side of said inner layer and said inner layer-facing side of said outer layer;
   (C) one or more materials in said reservoirs which are activated or released when said reservoirs are activated, wherein said materials are selected from the group consisting of a detection material, an inactivating agent, or a combination thereof;
   (D) a permanent, pressure and/or heat sensitive adhesive covering a first portion of the perimeter of said opposite side of said inner layer; and
   (E) a peelable release layer covering said adhesive;
   wherein the adhesion of (1) said adhesive to itself or to said opposite side of said inner layer is greater than the strength of (2) the joining of said outer layer to said inner layer.
30. A method of disposing of a transdermal patch, said method comprising:
   (A) placing said transdermal patch on said opposite side of said inner layer of an article as defined in item 29; and
   (B) contacting said adhesive covering at least a portion of said opposite side of said inner layer with a different portion of said opposite side of said inner layer to form a sealed pocket enclosing said transdermal patch.
31. An article comprising:
   (A) an outer layer having an inner layer-facing side and an opposite side;
   (B) an inner layer having an outer layer-facing side and an opposite side, said inner layer being joined to said outer layer, to form one or more closed material reservoir between outer layer-facing side of said inner layer and said inner layer-facing side of said outer layer, and wherein a first portion of said opposite side of said inner layer is joined to a second portion of said opposite side of said inner layer, to form a pocket between first and second portions of said opposite side of said inner layer, said pocket having a sealable opening; and
   (C) one or more materials in said reservoirs which are activated or released when said reservoirs are activated, wherein said materials are selected from the group consisting of a detection material, an inactivating agent, or a combination thereof.
32. An article comprising:
   The present invention further provides an article comprising:
      (A) a tear-resistant outer layer having an inner layer-facing side and an opposite side;
      (B) a tear-resistant inner layer having an outer layer-facing side and an opposite side,
         said inner layer being joined to said outer layer to form one or more closed material reservoirs between said outer layer-facing side of said inner layer and said inner layer-facing side of said outer layer, and
         a first portion of said opposite side of said inner layer joined to a second portion of said opposite side of said inner layer, to form a pocket between first and second portions of said opposite side of said inner layer;
      (C) one or more materials in said reservoirs which are released when said reservoir are opened, wherein said materials are selected from the group consisting of a detection material, an inactivating agent, or a combination thereof;
      (D) a sealable flap adapted to seal said opening;
      (E) a permanent pressure and/or heat sensitive adhesive covering at least a portion of said flap; and
      (F) a peelable release layer covering said adhesive;
      wherein the adhesion of (1) said adhesive to said opposite side of said outer layer is greater than the strength of (2) the joining of said outer layer to said inner layer.
33. A method of disposing of a transdermal patch, said method comprising:
   (A) placing said patch in said pocket of an article as defined in item 32; and
   (B) sealing said pocket to form a sealed pocket enclosing said transdermal patch.
34. An article comprising:
   (A) a medicament layer having a transfer side and an opposite side;
   (B) a first adhesive contained in said medicament layer or covering at least a portion of said transfer side of said medicament layer, wherein the adhesive is optionally heat sensitive;
   (C) an outer layer having a medicament layer-facing side and an opposite side, said medicament layer being joined to said outer layer, to form one or more closed material reservoir between said opposite side of said medicament layer and said medicament layer-facing side of said outer layer; and
   (D) one or more materials in said reservoir which are activated or released when said reservoir is activated or opened, wherein said materials are selected from the group consisting of a detection material, an inactivating agent, or a combination thereof.
35. An article comprising:
   (A) a tear-resistant medicament layer having an medicament transfer side and an opposite side and containing an opiate;
   (B) a first pressure and/or heat sensitive adhesive contained in or covering at least a portion of said medicament transfer side of said medicament layer;
   (C) a second pressure and/or heat sensitive adhesive, which may the same as or different from said first adhesive and which is permanent, covering at least a portion of the peri-perimeter of said medicament layer.
   (D) a tear-resistant outer layer having a medicament layer-facing side and an opposite side, said medicament layer being joined to said outer layer to form one or more closed material reservoir between said opposite side of said medicament layer and said medicament layer-facing side of said outer layer;
   (E) one or more materials in said reservoir which are released when said reservoirs are opened, wherein said materials are selected from the group consisting of a detection material, an inactivating agent, or a combination thereof;
   (F) a first peelable release layer covering said first adhesive; and
   (G) a second peelable release layer covering said second adhesive;
   wherein said second adhesive is prevented from adhering to skin while said first adhesive is adhering to said skin, and
   wherein the adhesion of (1) said second adhesive to itself or to said opposite side of said outer layer is greater than the strength of (2) the joining of said outer layer to said medicament layer.
36. A method of disposing of an article, said method comprising folding an article as described in item 35 so that said opposite side of said medicament layer is sealed with said second adhesive.
37. An article comprising:
   (A) a transfer side and an opposite side wherein the transfer side comprises a first region and a second region adjacent to said first region, wherein said first and second regions are separated by a membrane;
   (B) one or more materials disposed in said first region selected from the group consisting of a detection material, and inactivating agent, or a combination thereof, wherein said materials are activated or released when said first region is folded onto said second region;
   a medicament disposed in said second region.
38. An article of item 37, wherein the inner and outer layers are tear-resistant.
39. An article of item 37, wherein the material in the material reservoir is selected from the group consisting of an indelible dye, a biological inactivating agent, a magnetic inactivating agent, a mechanical inactivating agent, a cross-linking inactivating agent, or any combination of any of the foregoing.
40. An article of item 37, wherein the reservoir is activated by application of pressure, cutting, opening, puncturing, sealing, tearing, unsealing, or any combination of any of the foregoing.
41. An article of item 37, wherein the inactivating agent is selected from the group consisting of rat or human mu-opioid receptor, opioid neutralizing antibodies, a narcotic antagonist, irritating or dipphoric agents, or any combination thereof;
42. An article of item 37, wherein the adhesive comprises a permanent, pressure and/or heat sensitive adhesive.
43. An article of item 37, wherein the first portion of said opposite side of said inner layer comprises at least a portion of the perimeter of said opposite side of said inner layer.
44. An article of item 37, wherein the article further comprises (E) a release layer covering said adhesive, wherein said release layer is optionally peelable.
45. An article of item 37, wherein the adhesion of (1) said adhesive to itself or to said opposite side of said inner layer is greater than the strength of (2) the joining of said outer layer to said inner layer.
46. A container comprising (a) one or more material reservoirs sized to receive a transdermal dosage form; and (b) an adhesive capable of sealing a transdermal dosage form within the reservoir, wherein the adhesive is a heat activated adhesive that changes color and/or is activated when the article is subjected to elevated temperatures, thereby sealing the transdermal dosage form within the article.
47. A container of item 46 wherein the reservoir(s) comprises a flap covered at least in part by a permanent pressure and/or heat sensitive adhesive.
48. A container of item 46 wherein the container further comprises a peelable release layer covering the adhesive.
49. A container of item 46 further comprising a detection material and/or medicament inactivating agent in communication with the reservoir which is released when the reservoir is opened or revealed.
50. A container of item 46 wherein the container is tear-resistant.
51. A container of item 46 wherein the container further comprises a medicament-containing reservoir.
52. A container of item 51 wherein the container further comprises a medicament-containing reservoir and a detection material and/or inactivating agent-reservoir.
53. A container comprising (a) one or more material reservoirs sized to receive a transdermal dosage form; and (b) a means for sealing a transdermal dosage form within the reservoir.
54. A container of item 53 wherein component (b) is an adhesive that is a heat activated adhesive that changes color and/or is activated when the article is subjected to elevated temperatures, thereby sealing the transdermal dosage form within the article.
55. A container of item 53 wherein the reservoir(s) comprises a flap covered at least in part by a permanent pressure and/or heat sensitive adhesive.
56. A container of item 54 wherein the container further comprises a peelable release layer covering the adhesive.
57. A container of item 53 further comprising a means for detection and/or inactivation of unused medicament within a transdermal dosage form sealed within the reservoir, wherein said detection and/or inactivation means is in communication with the reservoir and said detection and/or inactivating means is released into the reservoir when the reservoir is opened or revealed.
58. A container of item 53 wherein the container is tear-resistant
59. A container of item 53 wherein the container further comprises a medicament-containing reservoir.
60. A container of item 59 wherein the container further comprises a medicament- containing reservoir and a detection material and/or inactivating agent-reservoir.

## Claims

1. Disposal system for transdermal dosage form, said disposal system including an outer layer (1) and an inner layer (3), which are joined by an adhesive (4) covering at least a first portion of the inner layer, wherein the combination of the inner and outer layers forms a closed reservoir (2) between the outer layer facing-side of the inner layer and the inner layer-facing side of the outer layer, and wherein the reservoir (2) includes at least one of a detection material and an inactivator that is released when the disposal system or transdermal dosage form is misused, the perimeter of the reservoir (2) has a thickness in the range of 12-100 microns, and the center of the reservoir (2) is thinner than the perimeter of the reservoir.

2. The disposal system according to claim 1, wherein the detection material is selected from the group consisting of a dye, a pigment, a fluorescent material or any combination thereof.

3. The disposal system according to claim 2, wherein the detection material is an indelible dye.

4. The disposal system according to any one of claims 1 to 3, wherein the inactivating agent is selected from the group consisting of a biological inactivating agent, a chemical inactivating agent, a denaturing inactivating agent, an electrical inactivating agent, a magnetic inactivating agent, a mechanical inactivating agent, a cross-linking inactivating agent, or any combination thereof.

5. The disposal system according to claim 4, wherein the inactivating agent is selected from the group consisting of rat or human µ-opioid receptor, opioid neutralizing antibodies, a narcotic antagonist, irritating or dipphoric agents, or any combination thereof.

6. The disposal system according to any one of claims 1 to 5, further comprising a pocket having a sealable opening and formed between first and second portions of the opposite side of the inner layer.

7. Use of a disposal system according to any one of claims 1 to 6 for disposing a transdermal dosage form.

8. Use according to claim 7, wherein the transdermal dosage form includes an opioid as the active agent.

9. Use according to claim 8, wherein the active agent is selected from the group consisting of fentanyl, buprenorphine, etorphine and related opioids of sufficient potency to allow transdermal usage, or any combinations thereof.

## Patentansprüche

1. Entsorgungssystem für transdermale Dosierformen, wobei das Entsorgungssystem eine Außenschicht (1) und eine Innenschicht (3) beinhaltet, die durch einen Klebstoff (4) verbunden sind, der zumindest einen ersten Teil der Innenschicht abdeckt, wobei die Kombination der Innen- und der Außenschicht einen geschlossenen Behälter (2) zwischen der der Außenschicht zugewandten Seite der Innenschicht und der der Innenschicht zugewandten Seite der Außenschicht bildet, und wobei der Behälter (2) mindestens eins von einem Detektionsmaterial und einem Inaktivator beinhaltet, das bzw. der freigesetzt wird, wenn das Entsorgungssystem oder die transdermale Dosierform missbraucht wird, wobei der Umfang des Behälters (2) eine Dicke im Bereich von 12-100 Mikrometern aufweist, und die Mitte des Behälters (2) dünner ist als der Umfang des Behälters.

2. Entsorgungssystem nach Anspruch 1, wobei das Detektionsmaterial ausgewählt ist aus der Gruppe bestehend aus einem Farbstoff, einem Pigment, einem Fluoreszenzmaterial oder einer beliebigen Kombination davon.

3. Entsorgungssystem nach Anspruch 2, wobei das Detektionsmaterial ein Permanentfarbstoff ist.

4. Entsorgungssystem nach einem der Ansprüche 1 bis 3, wobei das Inaktivierungsmittel ausgewählt ist aus der Gruppe bestehend aus einem biologischen Inaktivierungsmittel, einem chemischen Inaktivierungsmittel, einem denaturierenden Inaktivierungsmittel, einem elektrischen Inaktivierungsmittel, einem magnetischen Inaktivierungsmittel, einem mechanischen Inaktivierungsmittel, einem vernetzenden Inaktivierungsmittel oder einer beliebigen Kombination davon.

5. Entsorgungssystem nach Anspruch 4, wobei das Inaktivierungsmittel ausgewählt ist aus der Gruppe bestehend aus Ratten- oder humanem µ-Opioidrezeptor, Opioid-neutralisierenden Antikörpern, einem narkotischen Antagonisten, reizenden oder dipphoric Mitteln oder einer beliebigen Kombination davon.

6. Entsorgungssystem nach einem der Ansprüche 1 bis 5, weiter umfassend eine Tasche, die eine verschließbare Öffnung aufweist und zwischen ersten und zweiten Abschnitten der entgegengesetzten Seite der Innenschicht gebildet ist.

7. Verwendung eines Entsorgungssystems nach einem der Ansprüche 1 bis 6 zur Entsorgung einer transdermalen Dosierform.

8. Verwendung nach Anspruch 7, wobei die transdermale Dosierform ein Opioid als Wirkstoff beinhaltet.

9. Verwendung nach Anspruch 8, wobei der Wirkstoff ausgewählt ist aus der Gruppe bestehend aus Fentanyl, Buprenorphin, Etorphin und verwandten Opioiden ausreichender Wirksamkeit, um einen transdermalen Gebrauch zu ermöglichen, oder beliebigen Kombinationen davon.

## Revendications

1. Système jetable pour une forme galénique transdermique, ledit système jetable comprenant une couche externe (1) et une couche interne (3), qui sont reliées par un adhésif (4) recouvrant au moins une partie de la couche interne, la combinaison des couches interne et externe forme un réservoir fermé (2) entre le côté faisant face à la couche externe de la couche interne et le côté faisant face à la couche interne de la couche externe, et dans lequel le réservoir (2) comprend au moins l'un d'un produit de détection et un désactivateur qui est libéré lorsque le système jetable ou la forme galénique transdermique est mal utilisé, le périmètre du réservoir (2) a une épaisseur dans la fourchette de 12 à 100 microns, et le centre du réservoir (2) est moins épais le périmètre du réservoir.

2. Système jetable selon la revendication 1, dans lequel le produit de détection est choisi dans le groupe composé d'un colorant, d'un pigment, d'un produit fluorescent ou d'une quelconque combinaison de ceux-ci.

3. Système jetable selon la revendication 2, dans lequel le produit de détection est un colorant indélébile.

4. Système jetable selon l'une quelconque des revendications 1 à 3, dans lequel l'agent désactivateur est choisi dans le groupe composé d'un agent désactivateur biologique, d'un agent désactivateur chimique, d'un agent désactivateur dénaturant, d'un agent désactivateur électrique, d'un agent désactivateur magnétique, d'un agent désactivateur mécanique, d'un agent désactivateur de réticulation, ou d'une quelconque combinaison de ceux-ci.

5. Système jetable selon la revendication 4, dans lequel l'agent désactivateur est choisi dans le groupe composé d'un récepteur du µ-opioïde de rats ou d'humains, des anticorps neutralisant les opioïdes, d'un antagoniste narcotique, des agents irritants ou dipphoriques, ou d'une quelconque combinaison de ceux-ci.

6. Système jetable selon l'une quelconque des revendications 1 à 5, comprenant également une poche ayant une ouverture scellable qui est formé entre la première et la deuxième parties du côté opposé de la couche interne.

7. Utilisation d'un système jetable selon l'une quelconque des revendications 1 à 6, pour jeter une forme galénique transdermique.

8. Utilisation de la revendication 7, dans lequel la forme galénique transdermique comprend un opioïde comme agent actif.

9. Utilisation de la revendication 8, dans lequel l'agent actif est choisi dans le groupe composé du fontanyl, de la buprenoiphinE, de l'étrophine et des opioïdEs apparentés de puissance suffisante pour un usage transdermique, ou une quelconque combinaison de ceux-ci.
